# EUROPEAN PATENT SPECIFICATION

(11) **EP 2 306 194 B1**
(45) Date of publication and mention of the grant of the patent: **20.04.2016**
(21) Application number: 09797663.3
(22) Date of filing: 30.06.2009
(51) Int. Cl.: G01N 33/531, G01N 33/543, G01N 33/553, G01N 33/533

(54) **DEVELOPING SOLUTION FOR IMMUNOCHROMATOGRAPHY, AND MEASUREMENT METHOD USING SAME**
ENTWICKLUNGSLÖSUNG FÜR DIE IMMUNCHROMATOGRAPHIE UND MESSVERFAHREN DAMIT
SOLUTION DE DÉVELOPPEMENT POUR L'IMMUNOCHROMATOGRAPHIE, ET PROCÉDÉ DE MESURE L'UTILISANT

(30) Priority: 14.07.2008 JP 2008182630
(43) Date of publication of application: 06.04.2011
(73) Proprietor: Tanaka Kikinzoku Kogyo K.K., Chiyoda-ku Tokyo 100-6422 (JP)
(72) Inventor: ITOH, Daisuke, Hiratsuka-shi Kanagawa 254-0076 (JP)
(74) Representative: Raynor, Stuart Andrew
(86) International application number: PCT/JP2009/003010
(87) International publication number: WO 2010/007734

(56) References cited:
- EP-A1- 2 251 690
- DE-A1- 4 129 901
- JP-A- 5 133 956
- JP-A- 5 133 956
- JP-A- 6 109 725
- JP-A- 10 177 028
- JP-A- 10 177 028
- JP-A- 2002 148 266
- JP-A- 2003 344 406
- JP-A- 2004 301 684
- JP-A- 2005 291 780
- JP-A- 2006 145 256
- JP-A- 2007 322 310
- JP-A- 2008 104 450
- US-A1- 2006 216 695

## Description

### TECHNICAL FIELD

The present invention relates to a developing solution which constitutes a mobile phase for immunochromatography, which is one type of immune measurement methods. The invention also relates to a measurement method using the developing solution which can be used for detecting a substance to be detected contained in a biosample with high sensitivity.

### BACKGROUND ART

As an immune measurement method for detecting a substance to be detected consisting of a specific antigen or an antibody based on a reaction between an antigen and an antibody specific to the antigen, a method of binding through an immunological reaction the substance to be detected in a sample to an antibody or an antigen that is bound to particles for detection via sensitization treatment and measuring an aggregation state of the particles for detection produced therefrom is known as a simple method, and from the view point that the results can be determined by the naked eye in particular, it is a method typically used.

In addition, a method of binding an antigen or an antibody labeled with a label to a substance to be detected in a sample based on an immunological reaction and measuring the label in a bound state is known. A radioimmunoassay which uses a radioactive isotope, an enzyme immunoassay which uses an enzyme, and a fluorescence immunoassay which uses a fluorescent material as a label have been also adopted.

According to these immune measurement methods, a step of reacting a substance to be detected with an antibody, etc. which is labeled with a label and a step of separating the label which is in a binding state with a substance to be detected from the label which is not in a binding state with substance to be detected are required. As a method of performing these steps by applying the principle of chromatography in a system consisting of an immobile phase and a mobile phase which continuously flows in contact with the immobile phase, an immunochromatography is known.

Regarding the type of immune measurement, a sandwich type and a competitive type, etc. are known. As a typical example of a sandwich type based on immunochromatography, operations as follows are carried out for detecting a substance to be detected in a sample consisting of an antigen.
(1) By using an antibody which specifically binds to an antigen, i.e., a substance to be detected, as a reagent for immobilization and coating a predetermined region of a medium for chromatography with the reagent for immobilization in a predetermined shape, etc., a reaction site is formed on any area of a medium for chromatography.
(2) Meanwhile, by having an antibody which specifically binds to a substance to be detected as a detection reagent and labeling the detection reagent with a label such as an enzyme or sensitizing the detection reagent with a label such as an insoluble carrier, a labeled detection reagent is prepared.
(3) A developing solution which constitutes a mobile phase is developed, together with a sample containing a substance to be detected and the labeled detection reagent, on a medium for chromatography, which is an immobile phase.

According to the operations above, at a reaction site which is formed on the medium for chromatography, an antigen as a substance to be detected is bound to an antibody, i.e., an immobilization reagent immobilized on the reaction site, and therefore captured. Also, as a result of the reaction between an antigen and an antibody by the antibody as a labeled detection reagent, a sandwich type conjugate of three components, i.e., an immobilization reagent (immobilized antibody), a substance to be detected (antigen), and a detection reagent (labeled antibody) is formed at the corresponding reaction site, and according to indirect binding of a label to the reaction site when a substance to be detected is present in a sample, a certain signal will appear, thereby the detection of the substance to be detected can be carried out based on it.

As it is simple to operate and measurement can be made in a short period of time, the immunochromatography is widely used for clinical test and measurement test in a lab, etc. As a label for immunochromatography, an enzyme or an insoluble carrier is generally used. By using an insoluble carrier which does not require any special operations and can be visually detected (e.g., colloidal metal particles or pigmented latex particles, etc.) as a label, its use value as a simple detection method, which is a characteristic of immunochromatography, becomes even higher.

However, when a detection reagent is sensitized by using an insoluble carrier for a label, it is essential for the labeled detection reagent to clearly develop and move on top of a medium for chromatography without any aggregation and reach the reaction site. For such reasons, in a developing solution for immunochromatography, a dispersion stabilizer is suitably added to stabilize the dispersion state of a detection reagent which is labeled with an insoluble carrier. Examples of the dispersion stabilizer include a protein, a polysaccharide, and a surface active agent. Specific examples include gelatin, casein, bovine serum albumin, gum Arabic, dextran, starch, methyl cellulose, polylysine, polyproline, and polyethylene glycol. However, when such dispersion stabilizer is added to a developing solution, generally the viscosity of the developing solution increases, which cause a decrease in development rate. The decrease in development rate is problematic in that not only the measurement time is increased and reproducibility of measurement results is lowered but also a false positive reaction which is created by a non-specific adsorption of components other than a substance to be detected that is included in a sample is caused.

Furthermore, there is another problem that, when an insoluble carrier is used as a label, signals that are detected from a reaction site are weaker compared to a case in which an enzyme is used as a label. When an enzyme is used as a label, an insoluble material generated from an enzyme reaction accumulates at reaction site, yielding amplification of positive signal. As such, measurement with relatively high sensitivity can be achieved. On the other hand, when an insoluble carrier is used as a label, only a positive signal which depends on the number of the insoluble carriers captured at the reaction site can be obtained, and this problem is especially significant when an insoluble carrier having small volume is used.

In view of the problems described above, suggestions have been made to use a vinyl-based water-soluble polymer having oxygen atom-containing polar groups as a dispersion stabilizer which does not increase viscosity of a developing solution (see Patent Document 1), to add an ionic surface active agent or a glycine derivative to a test solution including a substance to be detected to prevent non-specific adsorption of components other than the substance to be detected (see Patent Document 2 and Patent Document 3), or to use a polymer having a group having phosphobetaine structure in a side chain as an agent for enhancing sensitivity (see Patent Document 4) etc.

Patent Document 5 provides a developing solution for immunochromatography comprising a polymer prepared from a phosphorylcholine monomer and an ethylenically unsaturated monomer such as methacrylic acid. Patent Document 6 provides an immune-chromatographic detecting element whereby serum albumin and a straight-chain water-soluble polymer such as polyvinyl pyrrolidone may be passed through a porous substance together with a substance to be detected and a labelled reagent. Patent Document 7 provides particles for immunochromatography that include magnetic materials dispersed in organic polymeric substances. Patent Document 8 provides an immunochromatography measurement method for measuring substances in papillary secretions.

### Prior Art Documents

### Patent Documents

[Patent Document 1] Japanese Patent Application Laid-Open (JP-A) No. 5-133956
[Patent Document 2] JP-A No. 2005-291783
[Patent Document 3] JP-A No. 2005-291780
[Patent Document 4] JP-A No. 2003-344413
[Patent Document 5] JP 2003 344406 A
[Patent Document 6] JP 2002 148266 A
[Patent Document 7] JP 2006 145256 A
[Patent Document 8] JP 10 177028 A

### SUMMARY OF THE INVENTION

### PROBLEMS TO BE SOLVED BY THE INVENTION

However, none of the conventional technologies was satisfactory as immunochromatography that is highly sensitive and is able to sufficiently detect even a low-concentration substance to be detected while controlling false positive reactions caused by non-specific reaction of components other than the substance to be detected contained in a sample. Moreover, when dispersion state of a detection reagent that is labelled with an insoluble carrier is sufficiently increased, an appropriate distance is maintained among the insoluble carriers, therefore making it impossible to obtain amplification of positive signals at the reaction site. As a result, it was very difficult to provide a measurement method having high sensitivity.

### MEANS FOR SOLVING THE PROBLEMS

As a result of intensive studies, the inventors of the present invention found that, for measurement of a substance to be detected using immunochromatography, even a low-concentration substance to be detected can be detected with high sensitivity while inhibiting a non-specific reaction by developing a substance to be detected and a detection reagent labeled with an insoluble carrier in the presence of a vinyl-based water-soluble polymer and a non-ionic surface active agent, and realized the invention accordingly. In particular, the detection reagents which are present in an appropriate dispersion state in the presence of a vinyl-based water-soluble polymer having oxygen atom- and nitrogen atom-containing polar groups are brought together to bind to each other having the surface active agent as a binding aid by preparing the mobile phase with a developing solution containing a vinyl-based water-soluble polymer having oxygen atom- and nitrogen atom-containing polar groups and a non-ionic surface active agent, and as a result, the positive signal that is visually detected from the reaction site is amplified, and a signal to noise (S/N) ratio which could not have been achieved before can be obtained, and therefore the invention was realized.

Specifically, the invention relates to a developing solution including a vinyl-based water-soluble polymer having oxygen atom- and nitrogen atom-containing polar groups and a non-ionic surface active agent, which is used for immunochromatography.

Further, the invention relates to immunochromatography methods in which the developing solution described above constitutes a mobile phase. Further, the invention relates to a detection kit for immunochromatography methods having a chromatography medium for immunochromatography methods and the developing solution of the invention as described above.

Still further, the invention relates to a dilution solution for a sample of an immunochromatography method comprising the developing solution of the invention.

More detailed description of the invention is as follows.
(1) A developing solution for immunochromatography using a detection reagent labeled with an insoluble carrier, wherein the developing solution comprises a vinyl-based water-soluble polymer having oxygen atom- and nitrogen atom-containing polar groups and a non-ionic surface active agent having HLB value of 13 to 18, wherein the developing solution amplifies positive signal.
(2) The developing solution according to the above (1), wherein the insoluble carrier is a colloidal metal particle.
(3) The developing solution according to the above (2), wherein the colloidal metal particle is a colloidal gold particle.
(4) The developing solution according to any one of the above (1) to (3), wherein the vinyl-based water-soluble polymer having oxygen atom- and nitrogen atom-containing polar groups is chosen from polyvinyl pyrrolidone, dimethylacrylamide/vinyl pyrrolidone copolymer (the copolymerization ratio of dimethylacrylamide is 50 mol% or less), vinyl alcohol/vinyl pyrrolidone copolymer (the copolymerization ratio of vinyl alcohol is 50 mol% or less), and vinyl acetate/vinyl pyrrolidone copolymer (the copolymerization ratio of vinyl acetate is 20 mol% or less).
(5) The developing solution according to any one of the above (1) to (4), wherein the non-ionic surface active agent having HLB value of 13 to 18 is chosen from polyoxyethylene alkyl ether, polyoxyethylene sorbitan fatty acid ester, polyoxyethylene p-t-octylphenyl ether and polyoxyethylene p-t-nonylphenyl ether.
(6) An immunochromatography method wherein the developing solution according to any one of the above (1) to (5) is used as a developing solution which constitutes a mobile phase, wherein the developing solution amplifies positive signal.
(7) A detection kit for an immunochromatography method comprising a chromatography medium for an immunochromatography method and the developing solution according to any one of the above (1) to (5), wherein the developing solution amplifies positive signal.
(8) A dilution solution for a sample of an immunochromatography method, comprising the developing solution according to any of the above (1) to (5).

### EFFECTS OF THE INVENTION

According to the invention, regarding immunochromatography using an insoluble carrier as a label, immunochromatography which is precise and highly sensitive and is able to sufficiently detect even a low-concentration substance to be detected while inhibiting false positive reactions caused by non-specific adsorption of components other than the substance to be detected contained in a sample can be provided. In particular, since the vinyl-based water-soluble polymer having oxygen atom- and nitrogen atom-containing polar groups that is present in the developing solution as a mobile phase can increase the stability of dispersion state of a detection reagent labeled with the insoluble carrier and also the insoluble carriers are appropriately brought together to bind to each other by the non-ionic surface active agent, the positive signal from the reaction site is amplified while a non-specific reaction is inhibited. Accordingly, it becomes possible to obtain a signal to noise (S/N) ratio which could not have been achieved before.

Moreover, because the developing solution of the invention can be used not only as a developing solution but also as a dilution solution for a sample, a dilution solution used for diluting a sample can be used as it is as a developing solution for immunochromatography. In particular, for a case in which a substance to be detected is an influenza virus antigen, it can be used not only as a dilution solution for a sample such as a fluid obtained by wiping a nasal cavity or a pharynx but also as a solution for dissolving a sample containing a substance to be detected, having excellent solubility for the virus antigen.

### MODES FOR CARRYING OUT THE INVENTION

Hereinafter, the present invention will be explained in detail.

### Medium for Chromatography

Material of the medium for chromatography which is used for the immunochromatography of the invention is not specifically limited, if it is an inactive medium that consists of microporous materials exhibiting capillary phenomenon, does not react with a detection reagent to be used, an immobilization reagent, or a substance to be detected, and has development rate which allows the obtainment of sufficient sensitivity during a short-term determination.

Examples of the medium for chromatography for the invention include fine particles of a ceramic such as silica, titania, zirconia, ceria, and alumina, or fine particles of an organic polymer, a woven or non-woven matrix, membrane, filter paper, glass fiber filter paper, fabric, and cotton that are made of polyurethane, polyester, polyethylene, polyvinyl chloride, polyvinylidene fluoride, nylon, and cellulose derivatives such as nitrocellulose or cellulose acetate. The fine particles may not be porous by themselves, but function as a medium for chromatography by producing voids between fine particles when they are in a filled state. Preferred examples include a membrane or a filter paper of cellulose derivatives or nylon, and a glass fiber filter paper. More preferred examples include a nitrocellulose membrane, a mixed nitrocellulose ester (mixture of nitrocellulose and cellulose acetate) membrane, a nylon membrane, and a filter paper.

The shape and size of the medium for chromatography which is provided for the implementation of the invention are not specifically limited, and those that are allowed in terms of actual operation and observation of a reaction result can be used. For simpler operation, it is preferable that a support made of plastics, etc. is formed on the back side of a medium for chromatography in which a reaction site is formed on the surface. The properties of this support are not specifically limited. However, when observation of a measurement result is made based on naked-eye determination, it is preferable that the support has a color that is distinguished from the color developed by the label. In general, it is preferably colorless or white.

### Reaction Site

On top of the medium for chromatography used in the invention, a reaction site in which a substance which specifically binds to a substance to be detected, for example an antibody, is immobilized as an immobilization reagent on a certain location is formed. As a method of immobilizing an immobilization reagent to the medium for chromatography, there is a method of immobilizing directly the immobilization reagent to the medium for chromatography by physical or chemical means and a method of binding physically or chemically the immobilization reagent to fine particles such as latex particles and indirectly immobilizing the fine particles by capturing them by the medium for chromatography.

The direct immobilization can be achieved by physical adsorption or covalent bonding. In general, when the medium for chromatography is a nitrocellulose membrane or a mixed nitrocellulose ester membrane, physical adsorption can be carried out. For the covalent bonding, bromine cyanide, glutaraldehyde, carbodiimide, and the like are generally used for activation of the medium for chromatography. Any of these methods can be used. As an indirect immobilization method, there is a method by which an immobilization reagent is bound to insoluble fine particles followed by immobilization to the medium for chromatography. Regarding the diameter of the insoluble fine particles, those having a size which can be captured by the medium for chromatography but cannot move can be selected. Preferably, they are the fine particles having average particle diameter of about 10 µm or less. Various particles that are used for an antigen-antibody reaction are known. These well-known particles can be also used for the invention. Examples thereof include fine particles of an organic polymer material such as organic polymer latex particles obtained by emulsification polymerization including polystyrene, styrene-butadiene copolymer, styrene-methacrylic acid copolymer, polyglycidyl methacrylate, and acrolein-ethylene glycol dimethacrylate copolymer, fine particles including gelatin, bentonite, agarose, and cross-linked dextran, inorganic oxides such as silica, silica-alumina, and alumina, and inorganic particles in which a functional group is introduced by a silane coupling treatment, etc. to an inorganic oxide. For the invention, the direct immobilization is preferable in terms of easiness of controlling sensitivity, etc. Moreover, various methods can be used for the immobilization of an immobilization reagent to the medium for chromatography. For example, various techniques using a micro syringe, a pen equipped with a control pump, and an ink jet printing can be used. Shape of the reaction site is not specifically limited, and immobilization can be made to have a circular spot, a line, a number, a letter, or a symbol such as + and - which elongates vertically along the development direction of the medium for chromatography.

To prevent a decrease in precision degree of analysis by non-specific adsorption after the immobilization of an immobilization reagent, if necessary, a blocking treatment can be carried out for the medium for chromatography according to a known method. In general, for the blocking treatment a protein such as bovine serum albumin, skim milk, casein, and gelatin is preferably used. After the blocking treatment, if necessary, washing may be carried out by using either one or combination of two or more surface active agents such as Tween20, Triton X-100 and SDS.

Moreover, if necessary, a sample addition site to add a sample containing a substance to be detected (e.g., a sample pad), a site for removing solid matters such as blood cells in a sample (e.g., a blood cell separation site), a developing solution addition site to add the developing solution, an absorption site in which the developing solution or a substance to be detected that is not captured at the reaction site is absorbed (e.g., an absorption pad), or a comparison site to verify the correct measurement can be further incorporated to the medium for chromatography. Materials for these sites are not specifically limited, if the sample solution or the developing solution can move according to a capillary phenomenon. In general, it is selected from many porous materials such as nitrocellulose membrane, filter paper, and glass fiber filter paper depending on the purpose, and arrangement can be made such that the material is linked via capillaries to the medium for chromatography to which the immobilization reagent is immobilized.

### Labels

The detection reagent used in the invention is a substance which specifically binds to a substance to be detected, for example an antibody, and it is labeled with a label. As a label for the detection reagent of immunochromatography, an enzyme and the like are generally used. However, from the viewpoint that it is suitable for naked-eye determination of the presence of a substance to be detected, an insoluble carrier is used as a label for the invention. According to the invention, a labeled detection reagent is prepared by sensitizing the insoluble carrier with a detection reagent.

As an insoluble carrier which is used as a label in the invention, a colloidal metal particle such as gold, silver, and platinum, a colloidal metal oxide particle such as iron oxide, a colloidal non-metal particle such as sulfur, a latex particle consisting of synthetic polymers, and others can be used.

Preferably, the insoluble carrier is a label which is suitable for visual determination of the presence of a substance to be detected and has a color for easy naked-eye determination. As the colloidal metal particles and colloidal metal oxide particles exhibit a specific natural color by themselves according to their particle size, their color can be used as a label. On the other hand, the latex particles consisting of synthetic polymers are white in natural state, and therefore cannot be used as a label by themselves. However, according to dyeing of the latex particles in an aqueous medium with an emulsion of a solution containing an oil-soluble dye using an organic solvent, they can be prepared to have desired color at desired concentration.

The latex particles which can be used as a label for the invention can be obtained by polymerization or co-polymerization of various monomers. Examples of the monomer include polymerizable unsaturated aromatics such as styrene, chlorostyrene, α-methylstyrene, divinyl benzene, and vinyl toluene, polymerizable unsaturated carboxylic acids such as (meth) acrylic acid, itaconic acid, maleic acid, and fumaric acid, polymerizable unsaturated carboxylic acid esters such as methyl (meth)acrylate, ethyl (meth)acrylate, n-butyl (meth)acrylate, 2-hydroxyethyl (meth)acrylate, glycidyl (meth)acrylate, ethylene glycol-di-(meth)acrylate ester, and tribromophenyl (meth)acrylate, unsaturated carboxylic acid amide, polymerizable unsaturated nitriles, vinyl halides, and conjugated dienes such as (meth) acrylonitrile, (meth) acrolein, (meth)acrylamide, N-methylol-(meth)acrylamide, methylene bis(meth)acrylamide, butadiene, isoprene, vinyl acetate, vinyl pyridine, N-vinyl pyrrolidone, vinyl chloride, vinylidene chloride, and vinyl bromide. These monomers are suitably selected according to the surface characteristics and specific gravity, etc. that are required for a label, and can be used singly or in combination of two or more.

Examples of the latex particles that are particularly preferred as a label by the invention include a copolymer of styrene and methacrylic acid and a copolymer of styrene and itaconic acid. As a polymerization initiator for the polymerization reaction to obtain such copolymers, persulfate salt and the like can be used. The average particle diameter of the latex particles used as a label is preferably within the range of 50 to 500 nm.

Meanwhile, examples of the colloidal metal particles and colloidal metal oxide particles that can be used as a label for the invention include colloidal gold particles, colloidal silver particles, colloidal platinum particles, colloidal iron oxide particles, and colloidal aluminum hydroxide particles. In particular, in terms of a suitable particle diameter, the colloidal gold particles and colloidal silver particles are preferred as the colloidal gold particles and colloidal silver particles exhibit red color and a yellow color, respectively. The average particle diameter of these colloidal metal particles is within 1 to 500 nm, preferably 10 nm to 150 nm, and more preferably 20 to 100 nm in which a particularly strong color is obtained.

Regarding these insoluble carriers, it is known that surface of both the latex particles and colloidal metal particles are negatively charged (see JP-A No. 5-133956). For example, on surface of the colloidal metal particles, anions derived from a reducing agent which is added during the manufacturing process are adsorbed on the surface, thus the aggregation among the particles is inhibited and the dispersion state is maintained. Furthermore, it is known that, when a surface active agent having low concentration which cannot neutralize the surface charges is added to the colloidal metal particles in this state, several particles are aggregated in chain form (see JP-A No. 2006-58781). Accordingly, for the immunochromatography of the invention, it is assumed that, a vinyl-based water-soluble polymer having oxygen atom- and nitrogen atom-containing polar groups and a non-ionic surface active agent are added to a developing solution which constitutes the mobile phase, several insoluble carriers that are captured at the reaction site on the medium for chromatography are aggregated, and as a result amplification of the positive signal observed from the reaction site is exhibited. In particular, with respect to the colloidal metal particles, it is assumed that, not only the signal intensity determined by the naked eye increases according to the increase in the number of particles accumulated at the reaction site by aggregation but also characteristics of light absorption spectrum of the particles change, and therefore more evident positive signal can be obtained from the reaction site. From the view point of having these advantages, the colloidal noble metal particles, in particular colloidal gold particles are preferred as a label of the invention.

As colloidal metal particles, when colloidal gold particles are used, for example, those that are commercially available can be used. Alternatively, the colloidal gold particles can be prepared by a general method, i.e., a method of reducing chloroauric acid with sodium citrate.

As a method of sensitizing the detection reagent used for the invention with the colloidal metal particles, a known method such as physical adsorption and chemical bonding can be used. For example, the detection reagent in which the colloidal gold particles are sensitized with an antibody is prepared by physical adsorption by adding an antibody to a solution in which gold particles are dispersed in a colloidal phase and blocking the surface of the particles to which no antibody is bound by adding a solution of bovine serum albumin.

For the actual implementation of the immunochromatography, the detection reagent labeled with an insoluble carrier can be applied by dispersing it in a developing solution which constitutes the mobile phase, or it can be applied by placing it on the development route of the mobile phase on a medium for chromatography, i.e., in an area between the terminal region to which the mobile phase of the medium for chromatography is applied and the reaction site. When it is present on the medium for chromatography, it is preferable to support the detection reagent so that the detection reagent is quickly dissolved in a developing solution and freely moves via capillary action. At the support site, in order to improve re-solubility of the insoluble carrier sensitized with a detection reagent, sugars such as sucrose, maltose, and lactose or sugar alcohols such as mannitol can be added and coated, or these substance can be coated in advance, etc. When the detection reagent is placed on top of the medium for chromatography by coating or drying, etc., it can be directly coated and dried on the medium for chromatography to which the immobilization reagent is immobilized, or after coating and drying on a separate porous material such as cellulose filter paper, glass fiber filter paper, and non-woven nylon to form a detection reagent-supported member, arrangement can be made such that the material is linked via capillaries to the medium for chromatography to which the immobilization reagent is immobilized.

### Substance to be Detected

The substance to be detected that is detected by the method of the invention is not specifically limited, if there is a substance which specifically binds thereto. Examples thereof include a protein, a peptide, a nucleic acid, a sugar (in particular, a sugar moiety of glycoproteins and a sugar moiety of glycolipids), and a complex sugar material. In the invention, the expression "specifically bind(s)" means that the binding is made based on an affinity of a biomolecule. As for the binding based on an affinity, binding between an antigen and an antibody, binding between a sugar and a lectin, binding between a hormone and a receptor, binding between an enzyme and an inhibitor, binding between complementary nucleic acids, and binding between a nucleic acid and a nucleic acid-binding protein can be exemplified. Thus, when the substance to be detected has an antigenic property, examples of a substance which specifically binds to the substance to be detected include a polyclonal antibody and a monoclonal antibody. In addition, when the substance to be detected is a sugar, examples of a substance which specifically binds to the substance to be detected include a lectin protein. Specific examples of the substance to be detected include a carcino embryogenic antigen (CEA), HER2 protein, prostate specific antigen (PSA), CA19-9, α-fetoprotein (AFP), immunosuppressive acidic protein (IAP), CA15-3, CA125, estrogen receptor, progesterone receptor, occult blood in stool, troponin I, troponin T, CK-MB, CRP, human chorionic gonadotropin (HCG), luteinizing hormone (LH), follicle stimulation hormone (FSH) , antibody against syphilis, influenza virus, human hemoglobin, clamidia antigen, A group β streptococcus antigen, HBs antibody, HBs antigen, rotavirus, adenovirus, albumin, and glycated albumin, but not limited thereto. Among these, the antigen which is solubilized by a non-ionic surface active agent is preferable. An antigen which forms a self-conjugate such as virus nuclear proteins is more preferable.

Examples of the sample containing the substance to be detected as described above include a biosample, i.e., whole blood, blood serum, blood plasma, urine, saliva, phlegm, fluid obtained by wiping a nasal cavity or a pharynx, body fluid, amniotic fluid, nipple discharge, tear, sweat, effusion fluid from a skin, and an extraction fluid from a tissue, a cell or feces. If necessary, these samples are treated to have a state in which the substance to be detected and the detection reagent or the immobilization reagent can easily achieve a specific binding reaction. The treatment method can be any one of a chemical treatment using various chemicals such as an acid, a base, and a surface active agent and a physical treatment by heating, stirring, and ultrasonication, etc., or both of them can be used. In particular, when a binding reaction of the substance to be detected and the detection reagent or the immobilization reagent is carried out by utilizing a region which is not generally exposed to the surface such as influenza virus NP antigen, it is preferable to carry out the treatment using a surface active agent. As for the surface active agent used under this purpose, a non-ionic surface active agent is preferably used considering the influence on a specific binding reaction, for example an antigen-antibody reaction. Moreover, if necessary, these samples may be diluted with the developing solution to allow the development on the medium for chromatography as an immobile phase.

For the actual implementation of the immunochromatography, application of the sample to the reaction site can be carried out by developing the sample solution on the medium for chromatography and allowing it to move chromatographically. In such case, the development of the sample solution can be carried out simultaneously with the development of the developing solution which constitutes the mobile phase, or it is also possible that the sample solution is developed first followed by development of the developing solution.

### Developing Solution

The developing solution used for the invention is a liquid which constitutes the mobile phase of the immunochromatography, and it moves along the surface of the medium for chromatography which is the mobile phase together with the sample containing a substance to be detected and a labeled detection reagent.

In addition, as the developing solution of the invention can be used not only as a developing solution but also as a dilution solution for a sample, the dilution solution used for the dilution of a sample can be used as it is as a developing solution for the immunochromatography.

### Vinyl-Based Water-Soluble Polymer

The developing solution used for the invention is characterized in that it contains a vinyl-based water-soluble polymer having oxygen atom- and nitrogen atom-containing polar groups. Examples of the vinyl-based water-soluble polymer having oxygen atom- and nitrogen atom-containing polar groups include a polymer having a structure unit in which double bond of a monomer is cleaved, wherein the monomer is a vinyl-based water-soluble monomer having oxygen atom- and nitrogen atom-containing polar groups such as (meth)acrylamide, dimethyl(meth)acrylamide, and vinyl pyrrolidone, more preferably a nonionic vinyl-based water-soluble monomer having oxygen atom- and nitrogen atom-containing polar groups. The polymer having a structure unit in which the double bond of vinyl pyrrolidone is cleaved is particularly preferred.

These vinyl-based water-soluble polymers may be those obtained by copolymerization with other vinyl-based monomer such as vinyl acetate and alkyl (meth)acrylate in an amount which will not negatively affect the effects of the invention, for example with the ratio of 50 mol% or less, preferably 30 mol% or less, and more preferably 15 mol% or less.

Specific examples of the preferred vinyl-based water-soluble polymer are polyvinyl pyrrolidone, dimethylacrylamide/vinyl pyrrolidone copolymer (the copolymerization ratio of dimethylacrylamide is 50 mol% or less), vinyl alcohol/vinyl pyrrolidone copolymer (the copolymerization ratio of vinyl alcohol is 50 mol% or less), and vinyl acetate/vinyl pyrrolidone copolymer (the copolymerization ratio of vinyl acetate is 20 mol% or less).

Molecular weight of the vinyl-based water-soluble polymer is generally 10,000 to 1,000,000, preferably 100,000 to 1,000,000, and more preferably 200,000 to 500,000. In addition, the concentration of the vinyl-based water-soluble polymer in the developing solution is preferably 0.01 to 5.0% by weight, and more preferably 0.1 to 2.0% by weight.

### Non-Ionic Surface Active Agent

The developing solution used in the invention contains a non-ionic surface active agent in addition to the vinyl-based water-soluble polymer having oxygen atom- and nitrogen atom-containing polar groups described above. As a non-ionic surface active agent, a polyoxyethylene-based surface active agent having HLB value of 13 to 18 can be used. Preferred examples of the polyoxyethylene-based surface active agent include polyoxyethylene alkyl ether, polyoxyethylene sorbitan fatty acid ester (trade name, "Tween" series), polyoxyethylene p-t-octylphenyl ether (trade name, "Triton" series), and polyoxyethylene p-t-nonylphenyl ether (trade name, "TritonN" series). Specific examples include, particularly for the "Tween" series, Tween20 (trade name, HLB value of 16.7), Tween40 (trade name, HLB value of 15.6), Tween60 (trade name, HLB value of 15.0) and Tween80 (trade name, HLB value of 14.9), particularly for the "Triton" series, Triton X-100 (trade name, HLB value of 13.5), Nonidet P-40 (trade name, HLB value of 13.1), TritonX-102 (trade name, HLB value of 14.6), TritonX-165 (trade name, HLB value of 15.8), and TritonX-405 (trade name, HLB value of 17.9), and particularly for the "TritonN" series, TritonN-101 (trade name, HLB value of 13.5), TritonN-111 (trade name, HLB value of 13.8), and TritonN-150 (trade name, HLB value of 15.0). The non-ionic surface active agent may be used singly or in combination of two or more. The content of the non-ionic surface active agent described above is not specifically limited. However, compared to the total weight of the composition, it is within the range of 0.01 to 10% by weight, and preferably 0.05 to 5% by weight.

The developing solution used in the invention generally contains water as a solvent and preferably contains a buffer agent in addition to the vinyl-based water-soluble polymer having oxygen atom- and nitrogen atom-containing polar groups and the non-ionic surface active agent. Preferred examples of the buffer agent include phosphate, trihydroxymethyl aminomethane, and Good's buffers. In addition, as an additional component, it may optionally contain a protein component such as bovine serum albumin (BSA) (the content is generally 0.01% by weight to 10% by weight).

According to an aspect of the invention, it is possible that the vinyl-based water-soluble polymer having oxygen atom- and nitrogen atom-containing polar groups and the non-ionic surface active agent are added in advance to the developing solution which constitutes a mobile phase on the medium for chromatography and the developing solution can be developed on the medium for chromatography. According to another aspect of the invention, it is possible that the vinyl-based water-soluble polymer having oxygen atom- and nitrogen atom-containing polar groups and the non-ionic surface active agent are placed on the development route of the mobile phase on the medium for chromatography, i.e., in an area between the terminal region to which the mobile phase of the medium for chromatography is applied and the reaction site, and they can be dissolved and added when a mobile phase is formed by the developing solution. According to still another aspect of the invention, it is possible that the non-ionic surface active agent is added to a sample solution containing a substance to be detected and the development of the sample solution is carried out simultaneously or before the development of the solution containing a vinyl-based water-soluble polymer having oxygen atom- and nitrogen atom-containing polar groups, so that the vinyl-based water-soluble polymer having oxygen atom- and nitrogen atom-containing polar groups and the non-ionic surface active agent can be added to the developing solution which constitutes the mobile phase on the development route of the mobile phase. In such case, it is not necessary that the solution containing a vinyl-based water-soluble polymer having oxygen atom- and nitrogen atom-containing polar groups contains the non-ionic surface active agent before it moves along the immobile phase. However, it may contain the non-ionic surface active agent of the invention.

Hereinafter, the present invention will be explained by the examples.

### [Example 1]

### 1. Production of a reaction site on the medium for chromatography

Using a 25x2.5 cm nitrocellulose membrane (trade name: HF120, manufactured by Millipore Corporation), anti-influenza B monoclonal antibody which is diluted to the concentration of 1.0 mg/mL with a phosphate buffer solution (pH 7.4) containing 5% by weight of isopropyl alcohol was coated by using an antibody coater (manufactured by BioDot Inc.), and then dried for 30 minutes at 50°C. After the drying, the nitrocellulose membrane was immersed in 200 mL phosphate buffer solution (pH 7.4) containing 0.5% by weight of casein (manufactured by Wako Pure Chemical Industries) for 30 minutes at 30°C to carry out the blocking. After the blocking, the membrane was washed with a washing solution containing 0.05% by weight of Tween20 and dried overnight at room temperature to produce the reaction site on the medium for chromatography.

### 2. Preparation of a solution containing the label

To 0. 5 mL of the suspension of gold colloid (manufactured by Tanaka Kikinzoku Kogyo K.K.: average particle diameter of 40 nm), 0.1 mL of anti-influenza B monoclonal antibody which is diluted to the concentration of 0.1 mg/mL with a phosphate buffer solution (pH 7.4) was added, and then maintained for 10 minutes at room temperature. Subsequently, 0.1 mL of the phosphate buffer solution (pH 7.4) containing 10% by weight of bovine serum albumin was added, stirred sufficiently, and then centrifuged with 8000xg for 15 minutes. After removing the supernatant, 0.1 mL of the phosphate buffer solution (pH 7.4) containing 1% by weight of bovine serum albumin was added to obtain the label solution.

### 3. Preparation of a medium for chromatography

The label solution as prepared above was evenly added to a pad made of glass fiber and dried with a vacuum drier to give a member for maintaining the developing reagent. Subsequently, to a base material consisting of a backing sheet, the medium for chromatography as prepared from the above, the member for maintaining the developing reagent, a sample pad used for the region to which the sample is added, and an absorption pad for absorbing developed sample and insoluble carrier were attached. Finally, it was cut by using a cutter to have the width of 5 mm, and as a result, the medium for chromatography was prepared.

### 4. Measurement

By using the medium for chromatography prepared from the above, the presence of influenza B virus in a sample was determined according to the following method. Specifically, the developing solution containing 0.5% by weight of Tween20, 0.6% by weight of polyvinyl pyrrolidone (PVP) K-90 (average molecular weight: 360,000), and 1.0% by weight of bovine serum albumin and Tris buffer solution (pH 8.0) containing 150 mM sodium chloride was employed as a negative test sample, and the developing solution to which influenza B virus obtained after the inactivation treatment has been added was employed as a positive test sample, and 150 µL of each sample was loaded on the sample pad of the medium for chromatography and developed. Fifteen minutes later, naked-eye determination was carried out. When the red line at the test line on the reaction site can be very clearly confirmed, it is indicated as "+++," when the red line can be clearly confirmed, it is indicated as "++," when the red line can be confirmed, it is indicated as "+, " when the red line can be confirmed but shows very weak color, it is indicated as "±", and when the red line cannot be confirmed, it is indicated as "-". The results are shown in Table 1.

### Comparative example 1

Except that the medium for chromatography prepared in the Example 1 is used and the developing solution not containing Tween20 is used, the measurement was carried out in the same manner as the Example 1. The results are shown in Table 1. The identical results were obtained even when twice the amount of PVP K-90 is used.

### Comparative example 2

Except that the medium for chromatography prepared in the Example 1 is used and the developing solution not containing PVP K-90 is used, the measurement was carried out in the same manner as the Example 1. The results are shown in Table 1. The identical results were obtained even when twice the amount of Tween20 is used.

### Comparative example 3

Except that the medium for chromatography prepared in the Example 1 is used and the developing solution not containing Tween20 and PVP K-90 is used, the measurement was carried out in the same manner as the Example 1. The results are shown in Table 1.

### Comparative example 4

Except that the medium for chromatography prepared in the Example 1 is used and 0.6% by weight of sodium carboxymethylcellulose (CMC·Na) (manufactured by FLUKA, viscosity 400-1000 mPa.s 2% in H₂O, 25°C) is used instead of PVP K-90 for the developing solution, the measurement was carried out in the same manner as the Example 1. The results are shown in Table 1.

### [Example 2]

Except that the medium for chromatography prepared in the Example 1 is used and 0.3% by weight of Triton X-100 is used instead of Tween20 for the developing solution, the measurement was carried out in the same manner as the Example 1. The results are shown in Table 1.

### Comparative example 5

Except that the medium for chromatography prepared in the Example 1 is used and 0. 6% by weight of polyethylene glycol (PEG) (manufactured by Wako Pure Chemical Industries, average molecular weight of 20,000) and 0.3% by weight of Triton X-100 are used instead of PVP K-90 and Tween20, respectively, for the developing solution, the measurement was carried out in the same manner as the Example 1. The results are shown in Table 1.

### Comparative example 6

Except that the medium for chromatography prepared in the Example 1 is used and 0.3% by weight of sodium cholate is used instead of Tween20 for the developing solution, the measurement was carried out in the same manner as the Example 1. The results are shown in Table 1.

### [Example 3]

Except that the medium for chromatography prepared in the Example 1 is used and 0.05% by weight of Tween20 and 0.3% by weight of Triton X-100 are used instead of 0.5% by weight of Tween20 for the developing solution, the measurement was carried out in the same manner as the Example 1. The results are shown in Table 1.

**[Table 1]**

| With blockinc | | | | | | | |
|---|---|---|---|---|---|---|---|
| | Water-soluble polymer | Surface active agent | Nuclear protein concentration originating from influenza B virus (ng/mL) | | | | |
| | | | 0 | 5 | 10 | 50 | S/N |
| Example 1 | PVP | Tween20 | - | + | + | +++ | +++ |
| Comparative example 1 | PVP | Not present | - | - | ± | ++ | ++ |
| Comparative example 2 | Not present | Tween20 | - | - | ± | ++ | ++ |
| Comparative example 3 | Not present | Not present | - | - | - | + | + |
| Comparative example 4 | CMC·Na | Tween20 | ++ | ++ | ++ | +++ | + |
| Example 2 | PVP | Triton X-100 | - | + | + | +++ | +++ |
| Comparative example 5 | PEG | Triton X-100 | ++ | ++ | ++ | +++ | + |
| Comparative example 6 | PVP | Na cholic acid | - | - | ± | + | + |
| Example 3 | PVP | Tween20 + Triton X-100 | - | + | ++ | +++ | +++ |

As a result of comparing the Examples 1 to 3 to the Comparative examples 1 to 3, it was evident that the nuclear protein originating from influenza B virus can be detected with high sensitivity in the presence of PVP, a vinyl-based water-soluble polymer having oxygen atom- and nitrogen atom-containing polar groups, and a non-ionic surface active agent. Meanwhile, when sodium carboxymethylcellulose (CMC·Na) or polyethylene glycol is used as a water-soluble polymer, non-specific adsorption was observed even from the negative test sample (Comparative examples 4 and 5). When sodium cholate, an ionic surface active agent, is used with PVP, it was impossible to achieve highly-sensitive detection (Comparative example 6). Thus, it is clear that by using the developing solution of the invention containing the vinyl-based water-soluble polymer having oxygen atom- and nitrogen atom-containing polar groups and the non-ionic surface active agent for immunochromatography a favorable S/N value can be obtained.

### [Example 4]

Except that the blocking and washing treatments are not carried out, the medium for chromatography was prepared in the same manner as the Example 1. Except that 0.05% by weight of Tween20 and 0.3% by weight of Triton X-100 are used instead of 0.5% by weight of Tween20 for the developing solution, the measurement was carried out in the same manner as the Example 1. The results are shown in Table 2.

**[Table 2]**

| Without blocking | | | | | | |
|---|---|---|---|---|---|---|
| | Water-soluble polymer | Surface active agent | Nuclear protein concentration originating from influenza B virus (ng/mL) | | | |
| | | | 0 | 2.5 | 5 | 10 |
| Example 4 | PVP | Tween20 + Triton X-100 | - | + | + | ++ |

Detection of the nuclear protein originating from influenza B virus in the presence of the vinyl-based water-soluble polymer having oxygen atom- and nitrogen atom-containing polar groups and the non-ionic surface active agent was achieved with high sensitivity, even when no blocking treatment was carried out for the medium for chromatography while non-specific adsorption of components other than the substance to be detected contained in a sample is inhibited. This means that a favorable S/N value can be obtained by simpler operations according to the present invention.

### [Example 5]

The medium for chromatography prepared in the Example 1 was used, PVPs with various molecular weight, i.e., 0.6% by weight of PVP K-30 (manufactured by Wako Pure Chemical Industries, average molecular weight of 40,000), 0.6% by weight of PVP K-60 (manufactured by Tokyo Kasei Kogyo, Co., Ltd., average molecular weight of 220,000) and 0.6% by weight of PVP K-90 (manufactured by Wako Pure Chemical Industries, average molecular weight of 360,000) were used, and 0.05% by weight of Tween20 and 0.3% by weight of Triton X-100 were used instead the measurement was carried out in the same manner as the Example 1. The results are shown in Table 3.

**[Table 3]**

| With blocking | | | | | | |
|---|---|---|---|---|---|---|
| | Water-soluble polymer | Surface active agent | Nuclear protein concentration originating from influenza B virus (ng/mL) | | | |
| | | | 0 | 5 | 7.5 | 10 |
| | PVP K-30 Average molecular weight 40000 | Tween20 + Triton X-100 | - | - | ± | + |
| Example 5 | PVP K-60 Average molecular weight 220000 | Tween20 + Triton X-100 | - | ± | + | + + |
| | PVP K-90 Average molecular weight 360000 | Tween20 + Triton X-100 | - | + | + + | + + |

From the Example above, the molecular weight of the vinyl-based water-soluble polymer having oxygen atom- and nitrogen atom-containing polar groups used in the invention was 100,000 to 1,000,000, and preferably 200,000 to 500,000.

### [Example 6]

The medium for chromatography prepared in the Example 1 was used, PVP K-90 (manufactured by Wako Pure Chemical Industries, average molecular weight of 360,000) with various use amount, i.e., 0.1% by weight, 0.3% by weight, 0.6% by weight, and 1.5% by weight, was used, and 0.05% by weight of Tween20 and 0.3% by weight of Triton X-100 were used instead of 0.5% by weight of Tween20 for the developing solution. Then, the measurement was carried out in the same manner as the Example 1. The results are shown in Table 4.

**[Table 4]**

| | With blocking | | | | | |
|---|---|---|---|---|---|---|
| | Water-soluble polymer | Surface active agent | Nuclear protein concentration originating from influenza B virus (ng/mL) | | | |
| | | | 0 | 5 | 7.5 | 10 |
| | PVP K⁻ 90 0.1% | Tween20 + Triton X-100 | - | - | ± | + |
| Example 6 | PVP K⁻ 90 0.3% | Tween20 + Triton X-100 | - | ± | + | ++ |
| | PVP K⁻ 90 0.6% | Tween20 + Triton X-100 | - | + | ++ | ++ |
| | PVP K⁻ 90 1.5% | Tween20 + Triton X-100 | - | + | ++ | ++ |

From the Example above, the use amount of the vinyl-based water-soluble polymer having oxygen atom- and nitrogen atom-containing polar groups used in the invention is 0.01 to 5% by weight, and preferably 0.1 to 2% by weight.

### [Example 7]

### 1. Production of a reaction site on the medium for chromatography

On a 25×2.5 cm nitrocellulose membrane (trade name: HF120, manufactured by Millipore Corporation), anti-human hemoglobin antibody which is diluted to the concentration of 1. 0 mg/mL with a phosphate buffer solution (pH 7.4) containing 5% by weight of isopropyl alcohol was coated using an antibody coater (manufactured by BioDot Inc.), and then dried for 30 minutes at 50°C. After the drying, the nitrocellulose membrane was immersed in 200 mL phosphate buffer solution (pH7.4) containing 0.5% by weight of casein (manufactured by Wako Pure Chemical Industries) for 30 minutes at 30°C to carry out the blocking. After the blocking, the membrane was washed with a washing solution containing 0.05% by weight of Tween20 and dried overnight at room temperature to produce the reaction site on the medium for chromatography.

### 2. Preparation of a solution containing the label

To 0.5 mL of the suspension of gold colloid (manufactured by Tanaka Kikinzoku Kogyo K.K., average particle diameter of 40 nm), 0.1 mL of anti-human hemoglobin monoclonal antibody which is diluted to the concentration of 0.1 mg/mL with a phosphate buffer solution (pH 7.4) was added, and then maintained for 10 minutes at room temperature. Subsequently, 0.1 mL of the phosphate buffer solution (pH 7.4) containing 10% by weight of bovine serum albumin was added, stirred sufficiently, and then centrifuged with 8000xg for 15 minutes. After removing the supernatant, 0.1 mL of the phosphate buffer solution (pH 7.4) containing 1% by weight of bovine serum albumin was added to obtain the label solution. The medium for chromatography was prepared in the manner as the Example 1.

### 3. Measurement

By using the medium for chromatography prepared from the above, the presence of human hemoglobin in a sample was determined according to the following method. Specifically, the developing solution containing 0.05% by weight of Tween20, 0.3% by weight of TritonX-100, 0.6% by weight of polyvinyl pyrrolidone (PVP) K-90 (average molecular weight: 360,000), 1.0% by weight of bovine serum albumin, and Tris buffer solution (pH 8.0) containing 150 mM sodium chloride was employed as a negative test sample, and the same developing solution to which human hemoglobin has been added was employed as a positive test sample, and 150 µL of each sample was loaded on the sample pad of the medium for chromatography and developed. Fifteen minutes later, naked-eye determination was carried out. When the red line at the test line on the reaction site can be very clearly confirmed, it is indicated as "+++," when the red line can be clearly confirmed, it is indicated as "++, " when the red line can be confirmed, it is indicated as "+, " when the red line can be confirmed but shows very weak color, it is indicated as "±," and when the red line cannot be confirmed, it is indicated as "-." The results are shown in Table 5.

**[Table 5]**

| With blocking | | | | | | |
|---|---|---|---|---|---|---|
| | Water-soluble polymer | Surface active agent | Human hemoglobin concentration (ng/mL) | | | |
| | | | Buffer only | 10 | 50 | 100 |
| Example 7 | PVP | Tween20 + Triton X-100 | - | ++ | +++ | +++ |

The immunochromatography which uses the developing solution of the invention enables the highly sensitive detection of the nuclear protein originating from influenza B virus and also human hemoglobin as a substance to be detected.

### INDUSTRIAL APPLICABILITY

The developing solution of the invention and the immunochromatography using it allow the highly sensitive detection of a substance to be detected contained in a biosample, and therefore they are widely used for the immune measurement method in clinical test, etc.

## Claims

1. A developing solution for immunochromatography using a detection reagent labeled with an insoluble carrier, wherein the developing solution comprises a vinyl-based water-soluble polymer having oxygen atom- and nitrogen atom-containing polar groups and a non-ionic surface active agent having HLB value of 13 to 18, wherein the developing solution amplifies positive signal.

2. The developing solution according to claim 1, wherein the insoluble carrier is a colloidal metal particle.

3. The developing solution according to claim 2, wherein the colloidal metal particle is a colloidal gold particle.

4. The developing solution according to any of claims 1 to 3, wherein the vinyl-based water-soluble polymer having oxygen atom- and nitrogen atom-containing polar groups is chosen from polyvinyl pyrrolidone, dimethylacrylamide/vinyl pyrrolidone copolymer (the copolymerization ratio of dimethylacrylamide is 50 mol% or less), vinyl alcohol/vinyl pyrrolidone copolymer (the copolymerization ratio of vinyl alcohol is 50 mol% or less), and vinyl acetate/vinyl pyrrolidone copolymer (the copolymerization ratio of vinyl acetate is 20 mol% or less).

5. The developing solution according to any of claims 1 to 4, wherein the non-ionic surface active agent having HLB value of 13 to 18 is chosen from polyoxyethylene alkyl ether, polyoxyethylene sorbitan fatty acid ester, polyoxyethylene p-t-octylphenyl ether and polyoxyethylene p-t-nonylphenyl ether.

6. An immunochromatography method wherein the developing solution according to any of claims 1 to 5 is used as a developing solution which constitutes a mobile phase, wherein the developing solution amplifies positive signal.

7. A detection kit for an immunochromatography method comprising a chromatography medium for an immunochromatography method and the developing solution according to any one of Claims 1 to 5, wherein the developing solution amplifies positive signal.

8. A dilution solution for a sample of an immunochromatography method, comprising the developing solution according to any of claims 1 to 5.

## Patentansprüche

1. Entwicklungslöstung für Immunchromatographie unter Verwenden eines Detektionsreagens, das mit einem unlöslichen Träger markiert ist, wobei die Entwicklungslösung ein vinylbasiertes wasserlösliches Polmyer mit sauerstoffatom- und stickstoffatomhaltigen polaren Gruppen und einen nichtionischen grenzflächenaktiven Stoff mit einem HLB-Wert vo 13 bis 18 umfasst, wobei die Entwicklungslöstung ein positives Signal verstärkt.

2. Entwicklungslösung nach Anspruch 1, wobei der unlösliche Träger ein kolloidaler Metallpartikel ist.

3. Entwicklungslösung nach Anspruch 2, wobei der kolloidale Metallpartikel ein kolloidaler Goldpartikel ist.

4. Entwicklungslösung nach einem der Ansprüche 1 bis 3, wobei das vinylbasierte wasserlösliche Polymer mit sauerstoffatom- und stickstoffatomhaltigen polaren Gruppen gewählt ist aus Polyvinylpyrrolidon, Dimethylacrylamid/Vinylpyrrolidon-Copolymer (das Copolymerisationsverhältnis von Dimethylacrylamid beträgt 50 mol% oder weniger, Vinylalkohol/Vinylpyrrolidon-Copolymer (das Copolymerisationverhältnis von Vinylalkohol beträgt 50 mol% oder weniger) und Vinylacetat/Vinylpyrrolidon-Copolymer (das Copolymerisationsverhältnis von Vinylacetat beträgt 20 mol% oder weniger).

5. Entwicklungslösung nach einem der Ansprüche 1 bis 4, wobei der nichtionische grenzflächenaktive stoff mit einem HLB-Wert von 13 bis 18 gewählt ist aus Polyoxyethylenalkylether, Polyoxyethylen-Sorbitan-Fettsäureester, Polyoxyethylen-p-t-octylphenylether und Polyoxyethylen-p-t-nonylphenylether.

6. Immunchromatographieverfahren, wobei die Entwicklungslösung nach einem der Ansprüche 1 bis 5 als Entwicklungslösung verwendet wird, die eine mobile Phase bildet, wobei die Entwicklungslösung ein positives Signal verstärkt.

7. Detektionsset für ein Immunchromatographieverfahren, welches ein Chromatographiemedium für ein Immunchromatographieverfahren und die Entwicklungslösung nach einem der Ansprüche 1 bis 5 umfasst, wobei die Entwicklungslösung ein positives Signal verstärkt.

8. Verdünnungslösung für eine Probe eines Immunchromatographieverfahrens, welche die Entwicklungslösung nach einem der Ansprüche 1 bis 5 umfasst.

## Revendications

1. Solution de développement pour l'immunochromatographie utilisant un réactif de détection marqué avec un support insoluble, laquelle solution de développement comprend un polymère hydrosoluble à base de vinyle comportant des groupes polaires contenant des atomes d'oxygène et des atomes d'azote et un tensioactif non ionique ayant une balance hydrophile-lipophile de 13 à 18, laquelle solution de développement amplifie le signal positif.

2. Solution de développement selon la revendication 1, dans laquelle le support insoluble est une particule de métal colloïdale.

3. Solution de développement selon la revendication 2, dans laquelle la particule de métal colloïdale est une particule d'or colloïdale.

4. Solution de développement selon l'une quelconque des revendications 1 à 3, dans laquelle le polymère hydrosoluble à base de vinyle comportant des groupes polaires contenant des atomes d'oxygène et des atomes d'azote est choisi parmi la polyvinylpyrrolidone, le copolymère de diméthylacrylamide/vinylpyrrolidone (le rapport molaire de copolymérisation du diméthylacrylamide est de 50 % ou moins), le copolymère d'alcool vinylique/vinylpyrrolidone (le rapport molaire de copolymérisation de l'alcool vinylique est de 50 % ou moins), et le copolymère d'acétate de vinyle/vinylpyrrolidone (le rapport molaire de copolymérisation de l'acétate de vinyle est de 20 % ou moins).

5. Solution de développement selon l'une quelconque des revendications 1 à 4, dans laquelle le tensioactif non ionique ayant une balance hydrophile-lipophile de 13 à 18 est choisi parmi l'éther alkylique de polyoxyéthylène, l'ester d'acide gras de polyoxyéthylène sorbitan, l'éther p-t-octylphénylique de polyoxyéthylène et l'éther p-t-nonylphénylique de polyoxyéthylène.

6. Procédé d'immunochromatographie dans lequel la solution de développement selon l'une quelconque des revendications 1 à 5 est utilisée en tant que solution de développement qui constitue une phase mobile, dans lequel la solution de développement amplifie le signal positif.

7. Kit de détection pour un procédé d'immunochromatographie comprenant un milieu chromatographique pour un procédé d'immunochromatographie et la solution de développement selon l'une quelconque des revendications 1 à 5, dans lequel la solution de développement amplifie le signal positif.

8. Solution de dilution pour un échantillon d'un procédé d'immunochromatographie, comprenant la solution de développement selon l'une quelconque des revendications 1 à 5.
